# EUROPEAN PATENT APPLICATION

(11) **EP 4 385 560 A1**
(43) Date of publication of application: **19.06.2024**
(21) Application number: 22213480.1
(22) Date of filing: 14.12.2022
(51) Int. Cl.: A61N 5/06

(54) **SCALP TREATMENT DEVICE**

(71) Applicant: Koninklijke Philips N.V., 5656 AG Eindhoven (NL)
(72) Inventor: LAM, Hiu Man, 5656AG Eindhoven (NL); LELIEVELD, Mark Johannes, 5656AG Eindhoven (NL); BROWN, Guy Anthony, 5656AG Eindhoven (NL); CORREIA, Kevin Mario, 5656AG Eindhoven (NL); NUIJS, Antonius Maarten, 5656AG Eindhoven (NL); VAN DER ZWAN, Eduard Antonius, 5656AG Eindhoven (NL); CHEUNG, Kit Man Lisa, 5656AG Eindhoven (NL)
(74) Representative: Philips Intellectual Property & Standards

(57) **Abstract**

A light treatment device is for application to the scalp for providing scalp treatment. It has cushions for cushioning the side of the head from a force applied to hold the device to the head. Each cushion comprises a front section, a rear section and a space between the front and rear sections. Treatment light is provided to the inside surface of the spaces between the front and rear sections of the cushions.

## Description

### FIELD OF THE INVENTION

This invention relates to a light treatment device, and more particularly to a light treatment device using light for the treatment of the scalp.

### BACKGROUND OF THE INVENTION

Light bio-stimulation has been recognized as a method for providing stimulation to the scalp which has beneficial and therapeutic effects in terms of reducing flakes from dandruff or treating dry scalp. Light treatment is also known for stimulating the natural growth of hair.

Low level laser light is believed to enhance the physiological state of the scalp and encourage hair growth when radiated onto the hair follicles on the scalp. Non-coherent light sources such as light emitting diodes (LED) have also been described in this context.

Wearable helmets and wearable bands are commercially available for providing scalp treatment. For example, a user may need to wear such a device for 30 to 60 minutes for a whole treatment, daily. Therefore, comfort is one of the main concerns for users.

Comfort is needed while also ensuring the wearable device fixes well to the head. In particular, a wearable device will need a sufficient clamping force that it does not fall off.

One type of known device is a slim and light headband. It treats the scalp over a relatively small area, so the weight of the device is low and it can easily be fixed on the head.

Another type of known device is a helmet, which can treat the whole head in one application. However, this makes the device bulky and costly. In the case of a helmet design, it is known to provide a cushion at the sides of the head, for example as disclosed in CN 103372264.

### SUMMARY OF THE INVENTION

The invention is defined by the claims.

According to examples in accordance with an aspect of the invention, there is provided a light treatment device for treating the scalp, comprising:
a head section arranged to cover a portion of a head of a user, wherein the portion covers between one third and two thirds of the area of the scalp;
a pair of extension pieces extending from sides of the head section each arranged to overlie a respective side of the head proximal to the ear, wherein the extension pieces are biased towards each other in use to provide a gripping force to the sides of the head;
a respective cushion attached to each extension piece for cushioning the side of the head from the force applied by the respective extension pieces, wherein each cushion comprises a front section, a rear section and a space between the front and rear sections; and
a lighting arrangement arranged to provide treatment light to an inside surface of the head section and to the inside surface of the spaces between the front and rear sections of the cushions.

This device is for treating only a portion of the scalp so it can be lightweight and therefore comfortable to use. The cushions spread the gripping force applied to the extension pieces to improve comfort.

A space is defined between the sections of the cushions where therapy light delivery takes place. This space may be aligned with the scalp area in front of the ears i.e. the side burn area. Thus, no scalp area is missed when the device is used. Dandruff or dry scalp often occur at the sideburn area.

Each cushion for example has a U shape with the side limbs of the U shape formed by the front and rear sections. The connecting part of the U-shape is for example at the bottom (i.e. the U is upright).

The device is preferably usable in different orientations for treatment of different scalp areas, wherein in at least one use orientation the spaces between the front and rear sections cover a side burn area.

A gripping band is for example used to provide the bias of the extension pieces towards each other. The gripping band is for example pivotably attached to the extension pieces. In this way, it can be rotated to an upright position to provide support for the device even when the device is oriented at the back of the head.

The gripping band is for example slidably attached to the extension pieces by a pin and slot arrangement to allow translational adjustment between the gripping band and the extension pieces. This may be used to enable adjustment of the holding force.

The lighting arrangement for example comprises an arrangement of laser diodes.

In one example, the device is for treatment of one or more of dandruff, dry scalp, psoriasis, seborrheic dermatitis, acne and eczema. For this purpose, the lighting arrangement may be for providing light with a wavelength in the range 400nm to 470nm. For example blue light around 453nm may be used.

In another example, the device is for promoting hair growth. For this purpose, the lighting arrangement may be for providing light with a wavelength in the range 620nm to 670nm. For example red light around 650nm may be used.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a light treatment device of the invention;
Fig. 2 shows the device of Fig. 1 in a first orientation; and
Fig. 3 shows the device of Fig. 1 in a second orientation.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a light treatment device for application to the scalp for providing scalp treatment. It has cushions for cushioning the side of the head from a force applied to hold the device to the head. Each cushion comprises a front section, a rear section and a space between the front and rear sections. Treatment light is provided to the inside surface of the spaces between the front and rear sections of the cushions.

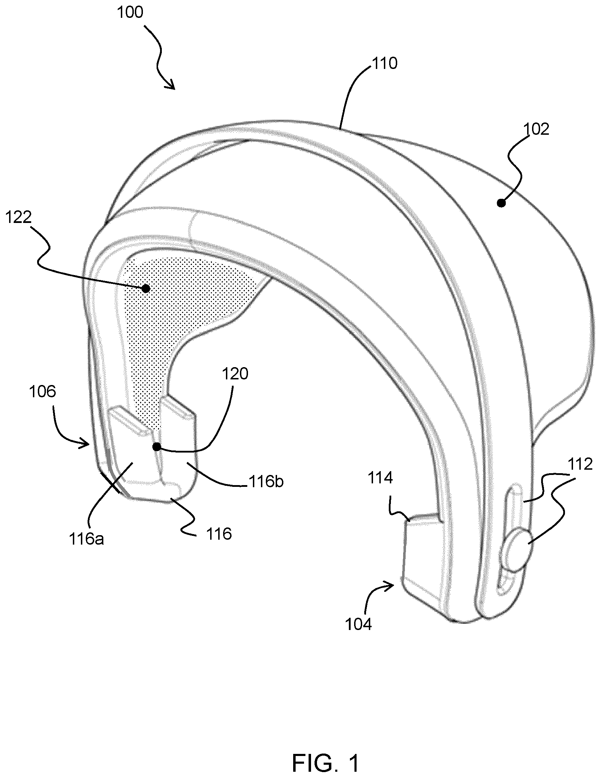

Fig. 1 shows a light treatment device 100 of the invention for promoting hair growth.

The device comprises a head section 102 arranged to cover a portion of a head of a user. The device is not a full helmet, so is intended to be used in multiple positions if the full scalp area is to be treated. The head portion thus covers between one third and two thirds of the area of the scalp (of a typical user). A typical average scalp area (averaging men and women) is around 700 cm². The device is thus lightweight and therefore comfortable to use.

There are first and second extension pieces 104, 106 extending downwardly (when the device has a default upright orientation as shown) from sides of the head section 102. They are each arranged to (partially) cover a respective side of the head proximal to the ear.

The extension pieces are biased towards each other in use to provide a gripping force to the sides of the head. This bias is for example provided by a gripping band 110.

The gripping band 110 is slidably attached to the outside of the extension pieces 104, 106 by a pin and slot arrangement 112 so that there is adjustability. This adjustability for example enables the clamping force applied by the gripping band to be adjusted. The width of the gripping band is different at different heights, so the clamping force applied can be varied depending on the use of a narrow position (tight) or wide position (loose) of the gripping band.

A cushion is attached to each extension piece for cushioning the side of the head from the force applied by the extension pieces. The first extension piece 104 has first cushion 114 and the second extension piece 106 has second cushion 116. The cushions spread the gripping force applied to the extension pieces to improve comfort.

As can be seen for cushion 116, each cushion comprises a front section 116a, a rear section 116b and a space 120 between the front and rear sections. The front and read sections are for example parallel linear cushion portions.

A lighting arrangement 122 is arranged to provide treatment light to an inside surface of the head section 102 and to the inside surface of the spaces 120 between the front and rear sections of the cushions. As shown, there is a continuous surface extending from the head section to the spaces between the cushions.

The space between the cushion sections is thus able to be used for light treatment. This space may be aligned (in one or more orientations of the device) with the hair growth area in front of the ears i.e. the side burn area. Thus, no scalp area is missed when the device is used.

Each cushion in this example thus has a U shape with the sections 116a, 116b defining the side limbs of the U shape and the connecting part of the U-shape at the bottom.

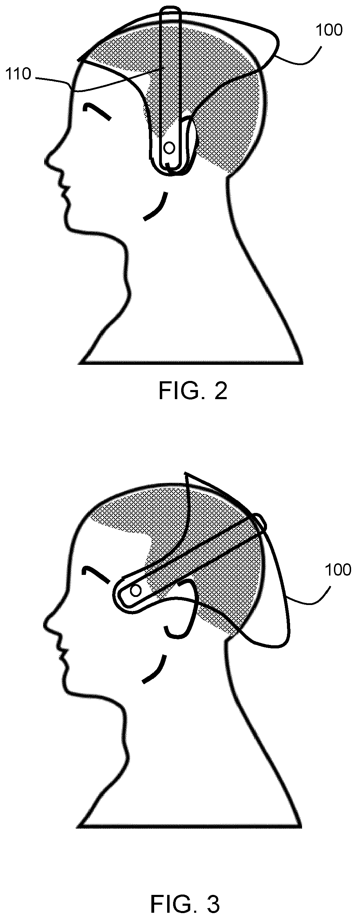

Fig. 2 shows the device 100 of Fig. 1 in a first orientation for treating a front area of the scalp. The device is stable on the head even with a small gripping force. The space between the cushion sections is aligned with the sideburn area.

Fig. 3 shows the device of Fig. 1 in a second orientation for treating a rear area of the scalp.

The gripping band 110 provides support to prevent the device falling from the head. The space between the cushion sections is again shown aligned with the side burn area.

The device is thus usable in different orientations for treatment of different scalp areas. In at least one use orientation the spaces between the front and rear sections cover a side burn area.

The lighting arrangement for example comprises an arrangement of laser diodes. Known and commercially available lighting systems may be used, for example laser diodes or LEDs.

In one example, the device is for treatment of dandruff or dry scalp. For this purpose, blue light may be used with a wavelength in the range 420nm to 470nm. For example, blue light around 453nm may be used.

In another example, the device is for promoting hair growth. For this purpose, red light may be used with a wavelength in the range 620nm to 670nm. For example, red low level laser light therapy (LLLT) light around 650nm may be used.

In other examples, the device may be used treatment of skin diseases on the scalp, such as psoriasis, seborrheic dermatitis, acne and eczema. For this purpose, light with a wavelength in the range 400nm to 470 nm has been shown to be effective. So, can claim 8 be expanded to include these diseases and can the range claimed in claim 9 be broadened, starting at 400 nm?

Of course any suitable wavelength may be used for a treatment of interest.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A light treatment device (100) for treating the scalp, comprising:
a head section (102) arranged to cover a portion of a head of a user, wherein the portion covers between one third and two thirds of the area of the scalp;
a pair of extension pieces (104, 106) extending from sides of the head section each arranged to overlie a respective side of the head proximal to the ear, wherein the extension pieces are biased towards each other in use to provide a gripping force to the sides of the head;
a respective cushion (114, 116) attached to each extension piece for cushioning the side of the head from the force applied by the respective extension pieces, wherein each cushion comprises a front section (116a), a rear section (116b) and a space (120) between the front and rear sections; and
a lighting arrangement (122) arranged to provide treatment light to an inside surface of the head section and to the inside surface of the spaces between the front and rear sections of the cushions.

2. The device of claim 1, wherein each cushion has a U shape with the side limbs of the U shape formed by the front and rear sections.

3. The device of claim, 1 or 2, wherein the device is usable in different orientations for treatment of different scalp areas, wherein in one use orientation the spaces between the front and rear sections cover a sideburn area.

4. The device of any one of claims 1 to 3, comprising a gripping band for providing the bias of the extension pieces towards each other.

5. The device of claim 4, wherein the gripping band is slidably attached to the extension pieces.

6. The device of claim 5, wherein the gripping band is slidably attached to the extension pieces by a pin and slot arrangement to allow translational adjustment between the gripping band and the extension pieces.

7. The device of any one of claims 1 to 6, wherein the lighting arrangement comprises an arrangement of laser diodes.

8. The device of any one of claims 1 to 7, configured for the treatment of one or more of:
dandruff;
dry scalp;
psoriasis;
seborrheic dermatitis;
acne; and
eczema.

9. The device of claim 8, wherein the lighting arrangement is for providing light with a wavelength in the range 400nm to 470nm.

10. The device of any one of claims 1 to 7, configured for promoting hair growth.

11. The device of claim 10, wherein the lighting arrangement is for providing light with a wavelength in the range 620nm to 670nm.
